# EUROPEAN PATENT SPECIFICATION

(11) **EP 4 312 579 B1**
(45) Date of publication and mention of the grant of the patent: **30.07.2025**
(21) Application number: 22720369.2
(22) Date of filing: 31.03.2022
(51) Int. Cl.: A23J 1/00, A23J 3/04, A23J 3/28, A23K 10/20, A23L 29/231, A23L 29/256, A23L 29/269, A23L 33/17, A23L 35/00, A23D 7/00, A23K 20/20, B01J 13/04

(54) **PROCESS AND SYSTEM FOR MAKING TEXTURED EDIBLE PROTEIN PRODUCT DERIVED FROM INSECT LARVAE OR WORMS**
VERFAHREN UND SYSTEM ZUR HERSTELLUNG EINES TEXTURIERTEN ESSBAREM PROTEINPRODUKTS AUS INSEKTENLARVEN ODER -WÜRMERN
PROCÉDÉ ET SYSTÈME POUR LA PRODUCTION D'UN PRODUIT PROTÉIQUE COMESTIBLE TEXTURÉ DÉRIVÉ DE LARVES D'INSECTES OU DE VERS D'INSECTES

(30) Priority: 31.03.2021 NL 2027887
(43) Date of publication of application: 07.02.2024
(73) Proprietor: Ynsect NL B.V., 3852AB Ermelo (NL)
(72) Inventor: HAGEMANS, Selke, 3513 GM UTRECHT (NL); AALBERS, Jan Jordan, 3841 KZ HARDERWIJK (NL); PETERS, Guus, 3722 JK BILTHOVEN (NL); VAN DE POLL, Jonkheer Theodoor Hendrik, 3446 HS WOERDEN (NL); ZANEN, Paul Sjerp, 3582 AW UTRECHT (NL); D'ANCONA, Coen Willem, 3437 DW NIEUWEGEIN (NL)
(74) Representative: Santarelli
(86) International application number: PCT/EP2022/058695
(87) International publication number: WO 2022/207866

(56) References cited:
- EP-A1- 3 262 958
- CN-A- 101 564 666
- CN-B- 107 223 812
- US-B2- 10 912 315
- D. SERP ET AL: "Characterization of an encapsulation device for the production of monodisperse alginate beads for cell immobilization", BIOTECHNOLOGY AND BIOENGINEERING, vol. 70, no. 1, 5 October 2000 (2000-10-05), pages 41 - 53, XP055042296, ISSN: 0006-3592, DOI: 10.1002/1097-0290(20001005)70:1<41::AID-BIT6>3.0.CO;2-U

## Description

The present invention relates to a process for producing a textured edible protein product derived from insect larvae or worms, to a system for continuously producing a textured edible protein product derived from insect larvae or worms.

### Background Art

As the world population grows, so does the need for proteins suitable for consumption. Cattle feedlots are the traditional source of proteins for consumption. However, they require a vast amount of money and energy to raise and feed, to remove waste and to keep them healthy. Worms and insects are a very suitable alternative to cattle feedstock. They offer an economical and sustainable solution to current issues with the production and distribution of proteins for consumption. Notably insect farming is much cheaper and requires much less energy than cattle farming. Much of this efficiency is a result of insects being exothermic. Insects obtain heat from the environment instead of having to create their own body heat like typical mammals do. Furthermore, feeding insects is cheap. Organic waste can for example feed large populations of insect larvae. Due to all these advantages, insect farming is gaining popularity. Besides being a good source of protein, insects also have a high nutritional value, probiotic potential and affordable price. Furthermore, they can have high concentrations of amino acids and certain vitamins such as vitamin B12, riboflavin, and vitamin A.

Many insects which have maggot and/or larval stages are suitable for insect farming. Mature larvae of different types of insects can be used as protein rich food for animals or humans. For example larvae from the Pachnoda marginata, also referred to as the Pachnoda butana, a beetle from the subfamily Cetoniina. Other examples may include:
- the *Alphitobius diaperinus,* a species of beetle in the family Tenebrionidae,
- the *Zophobas morio,* a species of darkling beetle, whose larvae are known by the common name superworm or zophobas,
- the mealworm beetle, *Tenebrio molitor,* a species of darkling beetle, the larvae being known as mealworms,
- the housefly, *Musca domestica,* is a fly of the suborder Cyclorrhapha, which larvae are known as maggots,
- Hermetia illucens, the black soldier fly, is a common and widespread fly,
- grasshoppers, insects of the order Orthoptera, suborder Caelifera,
- crickets family Gryllidae (also known as "true crickets"), are insects related to grasshoppers, well-known species of this family are Gryllus campestris (field cricket), Acheta domesticus (house cricket), and Gryllodes sigillatus (banded cricket),
- other insects such as *Bombyx mori, Achroia girsella, Schistocerca americana gregaria, Galleria mellonella, Locusta migratoria migratorioides.*

Most of these insects are holometabolous insects, i.e. including four life stages - as an embryo or egg, a larva, a pupa, and an imago or adult. The first stage is from the fertilization of the egg inside the mother insect until the embryo hatches. The insect starts as a single cell and then develops into the larval form before it hatches. The second stage lasts from hatching or birth until the larva pupates. In most species this mobile stage is worm-like in form, and these larvae are thus frequently referred to as "worms". The third stage is from pupation until eclosion. In preparation for pupation, the larvae of many species construct a protective cocoon of silk or other material, such as its own accumulated faeces. In this stage, the insect's physiology and functional structure, both internal and external, change drastically. Adult holometabolous insects usually have wings and functioning reproductive organs. In principle, the insects are harvested when the larvae are mature, i.e. near the end of the second stage, just before they turn into a pupa.

Culturally influenced aversion may result in some consumers being unwilling to eat an insect-based product, or even to feed such a product to an animal. Therefore, processes in which insects are abstracted to food products with a non-insect appearance are favourable. As a simple example, grinding larvae removes their insect appearance, but results in an untextured slurry which consumers find unappealing to use in food applications. EP 2953487 A1 for example therefore discloses a process in which insects after pulping are converted into nutrient streams, such as a fat-containing, an aqueous protein-containing and a solid-containing fraction. These nutrient streams may serve as ingredients in further feed and food preparations. The disclosed process is rather complex, as different products have to be separated, processed, packed and distributed. US10912315 discloses an edible textured insect protein product produced according to a process comprising processing the at least one insect to produce an insect milk by combining the at least one insect with an extraction buffer solution containing at least one of a monovalent salt, a divalent salt and a phosphate salt, and reducing the at least one insect into a plurality of insect particles of a desired particle size; adjusting the pH level of the insect milk; and heating the insect milk to coagulate the insect milk to form whey and at least one curd;wherein the extraction buffer solution contains the divalent salt at a concentration of about 0.25 to about 2% and wherein the edible textured insect protein product is substantially absent of exogenous gelling agents.wherein the edible textured insect protein product is substantially absent of exogenous gelling agents.

EP3262958 discloses a method for making a preparation of homogenized edible insect larvae to which binding agents are added in a ratio of 1 to 30 gram binding agent on a dry matter basis per 100 gram dead homogenised edible insect larva.

CN107223812 discloses a flake feed for fish fry cultivation, as well as a preparation method and application of the flake feed. The flake feed for fish fry cultivation consists of the following components: white fish meal, euphausia superba meal, fairy shrimp larval pulp (dry), sleeve-fish ointment, aquatic yeast, denaturated starch, sodium alginate, bacillus subtilis, vital gluten powder, lecithin, composite vitamin, mineral premix, fish oil and water.

D. SERP ET AL: "Characterization of an encapsulation device for the production of monodisperse alginate beads for cell immobilization",(BIOTECHNOLOGY AND BIOENGINEERING, part 70, nr. 1, 5 oktober 2000 (2000-10-05), pages 41-53) discloses the development and application of a new vibrating nozzle device, which has been commercialized by Inotech Encapsulation (Dottikon, Switzerland), including a detailed characterization of the technique in order to determine the potential for cell immobilization.

It would be advantageous to convert the entire insect into a single product, containing most to all of the nutrients of the insect. As indicated, pulped larvae as such are unappealing and difficult to use in food products, in large part due to the untextured properties of the slurry. The addition of gelling agents to the pulped larvae may alleviate this problem. A process is commonly known in which a solution of calcium ions is poured into a slurry of ground larvae, alginate and water. Upon contacting the calcium ions, the alginate immediately gelates together with the ground larvae. This results in large clumps and aggregates, in which the centre consists of ungelated slurry. This is undesirable. Instead, a flake like structure is desired in which substantially all hydrocolloid has been gelated with the metal cations. Therefore, during the addition of the calcium ion solution, the slurry should be vigorously stirred in a very specific fashion. Until now, the only solution was stirring by hand by a thereto trained operator. It goes without saying that such a process is unsuitable for upscaling, let alone for continuous production.

It is an objective of the present invention to alleviate one of the abovementioned disadvantages or at least to provide a useful alternative. It is a further objective of the present invention to provide a process for producing a textured edible protein product derived from insect larvae or worms, in which substantially all of the insect larvae or worms are converted into a single edible product. It is a further objective of the present invention to provide a process for producing a textured edible protein product which process is scalable. It is a further objective of the present invention to provide a process for producing a textured edible protein product in which process flakes of the textured edible protein product can be obtained without the interference of an operator. It is a further objective of the present invention to provide a process for producing a textured edible protein product which process is continuous. It is a further objective of the present invention to provide a textured edible protein product derived from insect larvae or worms.

### Description of the invention

In order to reach at least one of the objectives, the present invention provides a process for producing a textured edible protein product derived from insect larvae or worms. The process comprises the following steps:
a) reducing the insect larvae or worms in size to obtain a pulp,
b) mixing the pulp with a hydrocolloid that gelates with metal cations in aqueous solution to form a protein-hydrocolloid slurry,
c) injecting the protein-hydrocolloid slurry into an aqueous solution of a metal cation with a valency of at least 2 to form the textured edible protein product,
wherein in the injecting in step c) the protein-hydrocolloid slurry is jetted under pressure from outside of the aqueous solution of a metal cation with a valency of at least 2 into the aqueous solution at an oblique angle with respect to a liquid surface of the aqueous solution, thereby producing flakes of the textured edible protein product in the aqueous solution, in which flakes substantially all hydrocolloid has been gelated with the metal cations.

The present invention further provides a system for continuously producing a textured edible protein product derived from insect larvae or worms. The system comprises:
- a reaction vessel for holding an aqueous solution of a metal cation with a valency of at least 2,
- a reservoir for holding a protein-hydrocolloid slurry, said reservoir having an outlet in fluid communication with a pump and nozzle configured to inject a pressurized stream of the protein-hydrocolloid slurry from outside of the aqueous solution of a metal cation with a valency of at least 2 into the aqueous solution at an oblique angle with respect to a liquid surface of the aqueous solution,

- a controlled supply means for feeding metal cation into the reaction vessel for keeping a constant concentration of the metal cation in the aqueous solution,
- a separator for separating the edible protein product from the aqueous solution,
- a rinsing unit for rinsing the separated edible protein product,
- a dewatering press for dewatering and compacting the rinsed edible protein product.

In the process according to the invention, instead of adding the solution of the metal cation with a valency of at least 2 to the protein-hydrocolloid slurry, the slurry is jetted under pressure from outside of the aqueous solution of a metal cation with a valency of at least 2 into the aqueous solution at an oblique angle with respect to a liquid surface of the aqueous solution. Due to this process, flakes of the textured edible protein product are formed in the aqueous solution, in which flakes substantially all hydrocolloid has been gelated with the metal cations. In other words, it is due to the injection from outside of the aqueous solution of the metal cation onto the liquid surface of the aqueous solution and subsequently into the aqueous solution and at an oblique angle to the liquid surface of the aqueous solution, that flakes can be formed in which substantially all hydrocolloid has been gelated with the metal cations. Such flakes are not formed when injection is performed at a substantially perpendicular angle to the liquid surface, such as a bath surface, or the surface of a falling film, or when injection is performed directly into the solution of the metal cation, rather than from outside of the solution, i.e. not crossing the air-liquid interface. In the case of direct injection, strands of gelated material are formed instead of flakes. The strands may even have a liquid core in which the hydrocolloid has not been gelated. The hydrocolloid on the outside of such strands has been gelated with the metal cation, thereby forming a skin through which further metal cations could not reach the centre of the strand. Such strands do not have sufficient texture. In the case of a substantially perpendicular angle, undesirable blob like structures are formed, which may also have a liquid core in which the hydrocolloid has not been gelated. This as opposed to the flakes of the present invention, in which substantially all hydrocolloid has been gelated with the metal cations. In this respect "substantially all hydrocolloid has been gelated" can be read as "not comprising a liquid core".

When injection is substantially parallel to the liquid or bath surface, the slurry does not sufficiently cross the air-liquid interface and may bounce off the liquid surface. Even if the air-liquid interface is crossed, then flakes are not adequately formed. Instead, small particles are formed.

Although the mechanism is not known, it is speculated that the impact of the injected slurry into the liquid surface, thereby breaking the surface tension, and then through the aqueous solution causes a turbulence that brakes up the jet of injected slurry, thereby forming flakes of slurry in which the hydrocolloid gelates with the metal cation. The metal cations can penetrate into the centre of the flakes before a skin of gelated hydrocolloid has been formed on the outside of the flakes. The required turbulence is only created if the angle is oblique.

An additional advantage of the process and system according to the invention, is that the metal cation solution is physically separated from the opening, such as a nozzle, through which the slurry is discharged. This prevents the metal cation solution from entering the part of the setup in which the slurry resides, and thereby largely eliminates the risk of clogging of equipment due to prematurely gelated hydrocolloid. The invention is defined by the appended set of claims.

### Description of embodiments

Insect larvae or worms are rich in proteins and sometimes fats. Therefore, they represent a relatively high caloric value. Preferably, edible insect larvae or worms are used. More preferably, the insect larvae are larvae of flies, bugs, mosquitos, butterflies, moths, cicadas, termites, bees, ants, wasps, beetles, grasshoppers, or crickets.

Preferably, the insect larvae or worms are selected from the group constituted by *Tenebrio molitor, Hermetia illucens, Galleria mellonella, Alphitobius diaperinus, Zophobas morio, Blattera fusca, Tribolium castaneum, Rhynchophorus ferrugineus, Musca domestica, Chrysomya megacephala, Locusta migratoria, Schistocerca gregaria, Acheta domestica, Samia ricini* or mixtures thereof.

More preferably, the insect larvae are larvae of beetles. Preferably, the beetles belong to the families of the *Tenebrionidae, Melolonthidae, Dermestidae, Coccinellidae, Cerambycidae, Carabidae, Buprestidae, Cetoniidae, Dryophthoridae,* or mixtures thereof. Still more preferably, the beetles are *Tenebrio molitor, Alphitobius diaperinus, Zophobas morio, Tenebrio obscurus, Tribolium castaneum* and *Rhynchophorus ferrugineus,* or mixtures thereof. Still more preferably, the beetles are larvae of *Tenebrio molitor* or *Alphitobius diaperinus,* most preferably, *Alphitobius diaperinus.*

The insects or worms are preferably cultivated, e.g. in an insect farm. The cultivation allows to control and reduce the risks associated with diseases of insects and with the toxicity of insect-derived foodstuffs, e.g. due to the presence insecticides, in contrast to insects harvested in nature.

Cultivated worms and larvae as well as those harvested in nature comprise dirt, such as left-over feed, manure and shedded skin. In preparation of the process according to the invention, i.e. before step a), the harvested insect larvae or worms are therefore preferably cleaned, for example by washing them with water, preferably soft water. They may be rinsed or they may be submerged in water, preferably soft water, after which the excess water is drained. Any remaining water sticking to the insect larvae or worms after washing can be removed, but this is not necessary. A small amount of water sticking to the larvae or worms, or even some extra water added to the larvae or worms before executing step a) may even aid in the size reduction process. Typically, about 15 - 20 wt% of water, such as about 17 wt% with respect to the larvae weight sticks to the larvae after washing if no steps are taken to remove water after washing and draining the washing water. This corresponds to 13 - 17 wt%, such as 15% based on the weight of larvae and water combined. If extra water is added to the larvae or worms before size reduction step a), then preferably, the water is soft water. Preferably, before the size reduction step a), the larvae or worms are combined with 10 - 90 wt%, preferably 25 - 75 wt%, more preferably 35 - 65 wt%, most preferably 40 - 60 wt% of water, based on the weight of the larvae or worms (this water includes both extra added water and/or sticking water from washing). It is especially preferred that any water used in the process in or before steps a) and/or b) is soft water. The term soft water is known in the art. Soft water comprises no, or a minimalized amount of multivalent cations. It may be obtained by treatment of water with an ion exchange resin, by addition of softening salts such as a combination of tetrasodium pyrophosphate (TSPP) with NaCl, and other softening methods known in the art. This prevents early gelation of the hydrocolloid.

In a preferred embodiment, the insect larvae or worms are cleaned with water with a temperature of at least 60 °C, such as between 60 - 95 °C, for about 1 - 5 minutes, such as about 2 - 3 minutes, after which excess water is drained. This kills and cleans the larvae at the same time. In a preferred embodiment, the water is drained while continuously rinsing the larvae with fresh water. Alternatively, the larvae are submerged in the cleaning water, after which the water is drained.

In the process, one or more antioxidants may be added, for example to the cleaned insect larvae or worms, their pulp, or to the protein-hydrocolloid slurry. Many food and/or feed approved antioxidants are known. Antioxidants comprise synthetic antioxidants as well as natural antioxidants. Natural antioxidants are preferred, in large part due to their reputation as being from natural origin, and therefore "all natural", which is more appealing for consumers. Natural antioxidants include (with European additive and/or feed additive identification number between brackets) ascorbic acid (3a300, E300), tocopherols (E 306, 308, 309), tocopherol extracts from vegetable oils (1b306(i)), tocopherol-rich extracts from vegetable oils (delta rich), alpha-tocopherol (1 b307), tocotrienol, rosemary extract (E 392), gallic acid, carnosic acid, garlic extract, olive extract rich in phenolic compounds, and catechins-rich tea extract. Rosemary extract (i.e. rosemary oil) is especially preferred. The antioxidant may be added in an effective amount, for example from about 0.1 - 100 g/kg larvae, such as from 1 - 10 g/kg larvae.

In step a), the insect larvae or worms, optionally with water as mentioned earlier (sticking water and/or extra added water), are reduced in size to obtain a pulp. For example, they may be mashed, grinded, squashed, cut, mixed and/or milled to obtain the pulp. This results in a homogeneous starting material of viscous consistency. The reducing in size can conveniently be performed in a high shear mixer, although other suitable techniques can also be used. High shear mixers are well known in the art.

During the size reduction step, the particle size of the insect larvae or worms in the pulp is preferably reduced to less than 2 mm (the largest size to be determined using a microscope or using laser diffraction), more preferably less than 1 mm, even more preferably less than 0.5 mm. The smaller the particle size, the better, i.e. less grainy, the mouthfeel. The particle size can be controlled by selection of mixing time and speed. A skilled person can find suitable conditions in order to reach a desired particle size. In a preferred embodiment, the particle size is in the range of 0.1 - 1 mm.

Before, after or during mixing, the insect larvae or worms, the pulp or a pulp-water mixture may be heated at a temperature above 85°C, such as between 85 and 100 °C for at least 3 minutes, such as about 3 - 5 minutes, for pasteurization purposes. Pasteurization however may also be performed later in the process, such as during or after step b), or even after step c). For example, after formation, the flakes of the textured edible protein product may be heated at a temperature above 85°C, such as between 85 and 100 °C for at least 3 minutes, such as about 3 - 5 minutes. Preferably, pasteurization is performed before step b) in order to prevent caking of the equipment.

A solution of hydrocolloid in water, preferably soft water, may be prepared by dissolving the hydrocolloid in water, preferably with the aid of a mixer. The water in which the hydrocolloid is dissolved may provide the water for forming the aqueous solution in step b), or in case water was already added to the larvae or worms it provides extra water for forming the aqueous solution. Though the water does not need to be heated, heating the water to a temperature of between 50 - 100 °C, such as about 60 °C speeds up the dissolution process. A typical hydrocolloid concentration after dissolution is 3.5 - 4 % (w/v). The hydrocolloid that gelates with metal cations may be selected from pectin with a low methoxyl group content, Gellan gum and alginate; of these, sodium alginate is preferred.

The solution of hydrocolloid in water is subsequently mixed with the pulp. This forms the protein-hydrocolloid slurry. The mixing may again take place at a temperature of between 50 - 100 °C, such as about 60 °C. Preferably, the hydrocolloid that gelates with metal cations is present in a quantity of from 0.5 - 20 wt%, preferably of from 2 - 10 wt%, most preferably of from 4.5 - 5 wt% based on the weight of the larvae. Preferably, the protein-hydrocolloid slurry comprises from 10 - 75 wt% of larvae, more preferably from 20 - 50 wt%, even more preferably from 30 - 40 wt%, based on the combined weight of larvae, water and hydrocolloid. At lower amounts of larvae, the solution is not concentrated enough, and flakes are not adequately formed. At too high amounts of larvae, the solution is too viscous and cannot be adequately processed.

Depending on the hydrocolloid that gelates with metal cations which is used, it may be necessary to adapt the pH of the slurry prior to mixing with the solution of metal cations with a valency of at least 2. Preferably, the pH is in the range from 6 - 8.

Preferably, the aqueous solution of a metal cation with a valency of at least 2 contains soluble calcium or magnesium salts or mixtures thereof, preferably calcium chloride, calcium acetate, calcium lactate and/or calcium gluconate, such as calcium chloride, calcium acetate and/or calcium gluconate, most preferably calcium lactate and/or calcium chloride, still more preferably calcium chloride ; although other calcium or magnesium salts which are permitted for use in the food industry can also be used.

Calcium chloride is preferred because of the gelation speed. Use of salt other than calcium chloride generally decreases the speed of formation of the flakes. Calcium lactate is also particularly advantageous because it avoids formation of chloride sodium during the formation of flakes.

Upon preparation of the aqueous solution of a metal cation, it is advantageous to dissolve the metal cation with a valency of at least 2 in water at the same temperature as the protein-hydrocolloid slurry. The concentration of metal cations used in the gelation solution (i.e. the aqueous solution of a metal cation) is generally, for example in the case of calcium chloride, from 0.1 - 15% (w/v), expediently 0.1 - 5% (w/v) and preferably 0.5 - 5% (w/v). Generally, at high concentrations, the gelation process is faster, whereas at concentrations that are too low (i.e. less than 0.1 wt%), there is less cohesion within the flakes and/or a skin may be formed on the outside with ungelated hydrocolloid remaining inside of the flakes.

Preferably, the concentration of the metal cation with a valency of at least 2 in the aqueous solution is kept constant during injection step c). In this respect, constant means not varying more than 1% (w/v) higher or lower. Thus, if the value of the constant metal cation concentration is chosen to be 4% (w/v), then the concentration may vary from 3 - 5% (w/v).

Preferably, a concentrated metal cation solution is prepared and added to the aqueous solution of metal cation during the injection step c), for keeping the concentration in the metal cation solution constant during gelation of the product (flakes).

The protein-hydrocolloid slurry is then jetted under pressure from outside of the aqueous solution of a metal cation with a valency of at least 2 into the aqueous solution of metal cation at an oblique angle with respect to a liquid surface of the aqueous solution, thereby producing flakes of the textured edible protein product in the aqueous solution, in which flakes substantially all hydrocolloid has been gelated with the metal cations. Substantially all in this case can be read as meaning that more than 90 wt% of the hydrocolloid in the flakes has been gelated, preferably more than 95 wt%. Thus, the weight fraction of hydrocolloid in the flakes that is soluble in soft water is less than 10 wt%, preferably less than 5 wt%, based on the total weight of the hydrocolloid in the flakes.

By breaking the surface tension of the aqueous solution that contains metal cations with a valency of at least 2 and further entry of the slurry into the aqueous solution, flakes are formed, which, starting at their surface, are set through gelation of the hydrocolloid with metal cations. Due to the breaking of the surface tension and subsequent entry into the aqueous solution, the ratio of surface area vs. volume of the formed flakes is large enough that the flakes do not contain ungelated hydrocolloid. Without this breaking of the surface tension, the skin of gelated hydrocolloid and worm or larvae material formed on the gelated structures may form a barrier to diffusion of metal cations to the interior of the structures, thus creating slurry-filled blob like structures.

Thus, flakes of the textured edible protein product are formed in the aqueous solution, in which flakes substantially all hydrocolloid has been gelated with the metal cations.

In an embodiment, it is envisaged that the aqueous solution of the metal cation is present as a bath. The oblique angle (α) is then measured as the angle between the plane that is formed by the liquid surface of the aqueous solution of the bath and the smallest angle with the protein-hydrocolloid slurry jet at the point of impact.

In another embodiment, the aqueous solution of the metal cation is present in the form of a falling film. This film may be a free-falling film, but preferably the film flows/falls along a surface. The oblique angle of a falling film is measured as the angle between the plane that is formed by the liquid surface of the film and the angle with the protein-hydrocolloid slurry jet at the point of impact, with injection along the flow of liquid having an angle < 90°, and injection against the flow of liquid having an angle > 90 °. Injection along the flow of liquid is preferred.

Preferably, the oblique angle has a value of between 10 - 60 °, preferably between 15 - 50 °, more preferably between 20 - 40 °. This has been found the optimum angle. At too small angles, the slurry may bounce of the liquid surface and/or too small structures are formed, whereas at too high angles, blob like structures instead of flakes are formed, in which blob like structures not all hydrocolloid has gelated. The optimum angle has been found to be largely independent from the used pressure, throughput and nozzle diameter.

Preferably, jetting under pressure is performed through an opening, preferably a nozzle, more preferably a nozzle with a diameter of between 0.1 - 10 mm, preferably 1 - 5 mm.

When performed through a nozzle, preferably, the jetting under pressure is performed at a constant pressure in the nozzle, i.e. a pressure not varying more than 0.1 bar in either direction. Preferably, the value of the constant pressure is between 0.2 - 0.7 bar overpressure, more preferably between 0.3 - 0.6 bar overpressure, most preferably between 0.4 - 0.5 bar overpressure. Thus, if the value of the constant pressure is chosen to be 0.5 bar overpressure, then the pressure may vary from 0.4 - 0.6 bar overpressure.

Advantageously, the nozzle is a laminar flow nozzle, also named laminar nozzle.

Preferably, the protein-hydrocolloid slurry is jetted with a debit between 100 - 400 I/h, more preferably between 200 - 300 I/h, such as 250 I/h. When several nozzles are used, such a debit is preferably the debit of each nozzle.

Due to injection of the protein-hydrocolloid slurry into the aqueous metal cation solution (i.e. aqueous solution of a metal cation with a valency of at least 2), and subsequent gelation of the hydrocolloid with the metal cations in the solution, the remaining concentration of the metal cation in the aqueous solution in the reaction vessel is reduced. In order to counteract this depletion of ions, fresh metal cation may be fed to the aqueous metal cation solution in order to keep the metal cation concentration in the aqueous metal cation solution constant. This may for example be achieved by feeding a concentrated aqueous solution of a metal cation with a valency of at least 2 into the aqueous metal cation solution. In this respect, concentrated means a concentration which is higher than the concentration of the metal cation in the aqueous metal cation solution.

After formation, the flakes of the edible protein product may be separated from the aqueous solution, for example continuously, such as by a wire mesh conveyor, or batchwise, by scooping the edible protein product from the aqueous solution. The remaining aqueous solution comprises soluble proteins. If desired, the soluble proteins may be extracted from the remaining aqueous solution to form an additional nutrient stream.

After separation, preferably, the flakes of the edible protein product are preferably rinsed with water in order to wash out metal cations with a valency of at least 2 that have not yet reacted with hydrocolloid. Thorough rinsing with water, such as tap water, causes the metal cations that are present in excess to be rinsed out of the flakes.

After rinsing, preferably, the rinsed flakes of the edible protein product are dewatered, such as with a centrifuge, but preferably with a dewatering press, more preferably a dewatering screw press, and preferably to a moisture content of 50 - 90 wt%, more preferably 60 - 90 wt%.

The resulting material may be cut into adequately handleable pieces, and then packaged for transport. If not performed earlier in the process, the fibre-comprising food product according to the invention may be subjected to a treatment with a germicidal action after it has been packaged. A treatment with a germicidal action can then be selected from pasteurization, sterilization, and treatment with radiation.

### Brief description of the drawings

Fig. 1 represents a schematic picture of a system according to the invention.
Fig. 2 represents a schematic picture of the oblique angle α in different embodiments.

### Detailed description of drawings

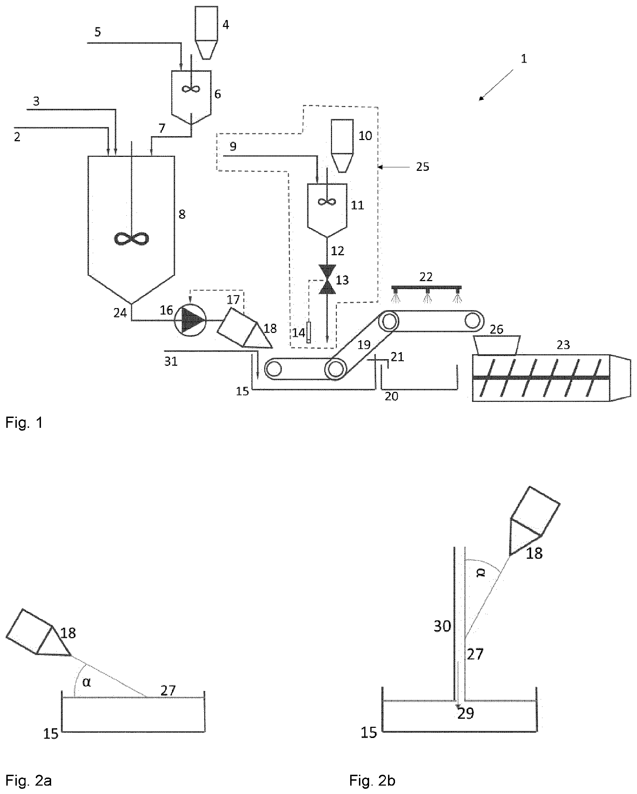

Fig. 1 represents a schematic picture of a system 1 for continuously producing a textured edible protein product derived from insect larvae or worms according to the invention. The system 1 comprises
- a reaction vessel 15 for holding an aqueous solution of a metal cation with a valency of at least 2,
- a reservoir 8 for holding a protein-hydrocolloid slurry, said reservoir 8 having an outlet 24 in fluid communication with a pump 16 and nozzle 18 configured to inject a pressurized stream of the protein-hydrocolloid slurry from outside of the aqueous solution of a metal cation with a valency of at least 2 into the aqueous solution at an oblique angle with respect to a liquid surface of the aqueous solution,
- a controlled supply means 25 for feeding metal cation into the reaction vessel 15 for keeping a constant concentration of the metal cation in the aqueous solution,
- a separator 19 for separating the edible protein product from the aqueous solution,
- a rinsing unit 22 for rinsing the separated edible protein product, and
- a dewatering press 23 for dewatering and compacting the rinsed edible protein product.

The reservoir 8 may be any type of reservoir suitable for holding the protein-hydrocolloid slurry. Advantageously, the reservoir 8 comprises mixing means and heating means. The reservoir 8 may further comprise an inlet 2 for the pulp, and an inlet 3 for soft water. The reservoir 8 may further comprise inlet 7 for the aqueous hydrocolloid solution, which may for example be fed by an aqueous hydrocolloid dispensing unit 6. Advantageously, the dispensing unit 6 comprises mixing means and heating means. The dispensing unit 6 is preferably fed by a soft water inlet 5 and a hydrocolloid powder dispensing unit 4.

The pump 16 and nozzle 18 preferably comprise a pressure control unit 17 for controlling the pressure of the pressurized stream of the protein-hydrocolloid slurry. The nozzle may be located above, diagonally above, or in the reaction vessel 15, as long as the nozzle opening is not in direct contact with the liquid when present in the reaction vessel 15. In a preferred embodiment, outlet 24 is in fluid communication with multiple combinations of pumps and nozzles, for simultaneously injecting multiple pressurized streams of the protein-hydrocolloid slurry, and thereby increasing the production capacity. Preferably, the system comprises at least one nozzle, preferably between 1 and 10 nozzles 18, more preferably between 2 and 8 nozzles 18, most preferably between 4 and 6 nozzles 18. If the system 1 comprises more than 1 nozzle 18, the nozzles 18 are implemented in parallel and preferably, the nozzles 18 share the same reaction vessel 15.

The reaction vessel 15 can be fed with the aqueous solution of a metal cation by controlled supply means 25. Controlled supply means 25 may for example comprise a reservoir 11 for holding a concentrated aqueous solution of a metal cation, which reservoir 11 further comprises an outlet 12, which is controlled by an ion sensor 14 in communication with valve 13. Advantageously, the reservoir 11 comprises mixing means and heating means. The reservoir 11 may further comprise a water inlet 9, which may be a tap water inlet, and a solid metal cation dispensing unit 10. When the ion sensor 14 detects that the ion level falls below a setpoint value, it may cause valve 13 to open and allow a sufficient amount of concentrated aqueous solution of metal cation to supplement the metal cations in the reaction vessel 15 through outlet 12.

The reaction vessel 15 advantageously comprises an outlet 21 for draining excess aqueous solution into drain unit 20. The reaction vessel 15 furthermore advantageously comprises an inlet 31 for soft water.

In a preferred embodiment, reaction vessel 15 comprises a means for creating a falling film of the aqueous solution of metal cation. The falling film may be free standing, or flowing along a surface 30. Surface 30 may be a vertical surface, or it may be inclined, preferably at an angle less than 90 °. The means for creating a falling film may for example include a pump and vertical or inclined surface. In a particularly advantageous embodiment, the falling film falls onto separator 19.

The separator 19 for separating the edible protein product from the aqueous solution may for example be a wire mesh conveyor. However, the separator can also be a paddle wheel or any other suitable separating means. The rinsing unit 22 may comprise one or multiple nozzles fed by a water supply in order to rinse the separated edible protein product.

The rinsing unit 22 is advantageously located above drain unit 20.

The inlet 26 of the dewatering press 23 is advantageously fed by the separator 19.

The dewatering press 23 may for example be a cheese press. Cheese presses are known in the art. However, preferably the dewatering press is a dewatering screw press. Dewatering screw presses are known in the art. They are slow moving devices that accomplish dewatering by continuous gravitational drainage. The screw press squeezes the material against a screen or filter and the liquid is collected through the screen.

Such a press usually consists of an inlet opening, a transport and press screw, a press section and an outlet. For wet materials, the press may be fitted with a sieve to drain the water. The screw transports the material to dewater from the inlet opening into to the press area. At the location of the press area, the screw blade is preferably adapted for compacting, that is, the screw blade may e.g. welded with a wear-resistant layer and the pitch is smaller than in the conveying part. The outlet is equipped with one or more controlled valves (e.g. pneumatically) with which the discharge pressure can be set. The supplied material to dewater is put under an increasing pressure by the rotating screw conveyor, which reduces the volume. The outlet valves open further and further when the set discharge pressure is reached and the compressed roll of material is slowly expelled.

The system 1 is adapted to perform the process for producing a textured edible protein product derived from insect larvae or worms according to the invention. Thus, this system advantageously presents the advantageous and preferred features and embodiments disclosed in the process.

Fig. 2a depicts the oblique angle α of the nozzle 18 with respect to the liquid surface 27 of the liquid in reaction vessel 15.

Fig. 2b depicts the oblique angle α of the nozzle 18 with respect to the liquid surface 27 of a liquid film which flows in direction 19 along surface 30 into the reaction vessel 15.

### Experiment

Larvae of *Alphitobius diaperinus* were harvested (23 kg) and submerged in water with a temperature of between 55 and 60 °C for a maximum of 3 minutes to simultaneously wash and kill the larvae, after which the larvae were separated from the water. Any water still sticking to the larvae after separation was not removed. The larvae were combined with 10 kg of soft water from a soft water supply, and subsequently, the larvae were reduced to a size of about 0.5 mm with a blender, thereby forming a pulp.

Then, 30 g of rosemary oil was added to the pulp, and further mixing was performed with the blender.

Alginate (1125 g = 48,9 g/kg of larvae) was dissolved in 36 kg of soft water of about 80 °C. The warm alginate solution and pulp were combined and mixed by stirring at a temperature of about 60 °C to form a slurry.

In a bath, 250 g of CaCl2 was dissolved in 10 kg of water of 90 °C at a concentration of about 2.5% w/v. The alginate-larvae slurry was fed to a pump which was connected to a nozzle with a diameter of about 2 mm and sprayed into the bath under an angle of 30 ° with an overpressure of about 0.5 bar. Flakes of the protein product were immediately formed upon impact with the CaCl2 solution. After all of the slurry was gelated, the solution was heated to above 90 °C for 3 minutes for pasteurization. Subsequently, the flakes were collected and washed twice with cold water. Dewatering was performed with a cheese press, and the resulting blocks of product were cut, vacuum-packed and stored in the freezer.

## Claims

1. Process for producing a textured edible protein product derived from insect larvae or worms, the process comprising the following steps:
a) reducing the insect larvae or worms in size to obtain a pulp,
b) mixing the pulp with a hydrocolloid that gelates with metal cations in aqueous solution to form a protein-hydrocolloid slurry,
c) injecting the protein-hydrocolloid slurry into an aqueous solution of a metal cation with a valency of at least 2 to form the textured edible protein product,
wherein in the injecting in step c) the protein-hydrocolloid slurry is jetted under pressure from outside of the aqueous solution of a metal cation with a valency of at least 2 into the aqueous solution at an oblique angle with respect to a liquid surface of the aqueous solution, thereby producing flakes of the textured edible protein product in the aqueous solution, in which flakes substantially all hydrocolloid has been gelated with the metal cations.

2. Process according to claim 1, wherein the oblique angle has a value of between 10 - 60 °, preferably between 15 - 50 °, more preferably between 20 - 40 °.

3. Process according to claim 1 or 2, wherein jetting under pressure is performed through an opening, preferably a nozzle, more preferably a nozzle with a diameter of between 0.1 - 10 mm, most preferably 1 - 5 mm.

4. Process according to any one of the previous claims, wherein the hydrocolloid that gelates with metal cations is sodium alginate.

5. Process according to any one of the previous claims, wherein the aqueous solution of a metal cation with a valency of at least 2 contains soluble calcium or magnesium salts or mixtures thereof.

6. Process according to any one of the previous claims, wherein the concentration of the metal cation in the aqueous solution is kept constant.

7. Process according to any one of the previous claims, wherein the hydrocolloid that gelates with metal cations is present in a quantity of from 0.5 - 20 wt%, preferably of from 2 - 10 wt%, most preferably of from 4.5 - 5 wt% based on the weight of the larvae.

8. Process according to any one of the previous claims, wherein the protein-hydrocolloid slurry comprises from 10 - 75 wt% of larvae, more preferably from 20 - 50 wt%, even more preferably from 30 - 40 wt%, based on the combined weight of larvae, water and hydrocolloid.

9. Process according to any one of the previous claims, wherein the aqueous solution of a metal cation with a valency of at least 2 is present as a bath and the liquid surface of the aqueous solution is the bath surface, or wherein the aqueous solution of a metal cation with a valency of at least 2 is present as a falling film, and the liquid surface of the aqueous solution is the surface of the falling film.

10. Process according to any one of the previous claims, wherein the flakes of the edible protein product are separated from the aqueous solution.

11. Process according to any one of the previous claims, wherein the flakes of the edible protein product are rinsed with water to remove excess metal cations.

12. Process according to any one of the previous claims, wherein the rinsed flakes of the edible protein product are dewatered, preferably with a dewatering press, more preferably a dewatering screw press, and preferably to a moisture content of 50 - 90 wt%, more preferably 60 - 90 wt%.

13. System (1) for continuously producing a textured edible protein product derived from insect larvae or worms the system comprising:
- a reaction vessel (15) for holding an aqueous solution of a metal cation with a valency of at least 2,
- a reservoir (8) for holding a protein-hydrocolloid slurry, said reservoir (8) having an outlet (24) in fluid communication with a pump (16) and nozzle (18) configured to inject a pressurized stream of the protein-hydrocolloid slurry from outside of the aqueous solution of a metal cation with a valency of at least 2 into the aqueous solution at an oblique angle with respect to a liquid surface of the aqueous solution,
- a controlled supply means (25) for feeding metal cation into the reaction vessel (15) for keeping a constant concentration of the metal cation in the aqueous solution,
- a separator (19) for separating the edible protein product from the aqueous solution,
- a rinsing unit (22) for rinsing the separated edible protein product,
- a dewatering press (23) for dewatering and compacting the rinsed edible protein product.

14. System according to claim 13, wherein the outlet (24) is in fluid communication with multiple combinations of pumps and nozzles, for simultaneously injecting multiple pressurized streams of the protein-hydrocolloid slurry.

## Patentansprüche

1. Verfahren zur Herstellung eines strukturierten essbaren Proteinprodukts aus Insektenlarven oder -würmern, wobei das Verfahren die folgenden Schritte umfasst:
a) Zerkleinern der Insektenlarven oder -würmer, um einen Zellstoff zu erhalten,
b) Mischen des Zellstoffs mit einem Hydrokolloid, das mit Metallkationen in wässriger Lösung geliert, um einen Protein-Hydrokolloidschlamm zu bilden,
c) Injizieren des Protein-Hydrokolloidschlamms in eine wässrige Lösung eines Metallkations mit einer Valenz von mindestens 2, um das strukturierte essbare Proteinprodukt zu bilden,
wobei beim Injizieren in Schritt c) der Protein-Hydrokolloidschlamm unter Druck von außerhalb der wässrigen Lösung eines Metallkations mit einer Valenz von mindestens 2 in die wässrige Lösung in einem Schrägwinkel zu einer flüssigen Oberfläche der wässrigen Lösung gespritzt wird, wodurch Flocken des strukturierten essbaren Proteinprodukts in der wässrigen Lösung erzeugt werden, wobei in den Flocken im Wesentlichen das gesamte Hydrokolloid mit den Metallkationen geliert worden ist.

2. Verfahren nach Anspruch 1, wobei der Schrägwinkel einen Wert zwischen 10 - 60 °, vorzugsweise zwischen 15 - 50 °, besser zwischen 20 - 40 ° aufweist.

3. Verfahren nach Anspruch 1 oder 2, wobei das Strahlen unter Druck durch eine Öffnung, vorzugsweise eine Düse, bevorzugter eine Düse mit einem Durchmesser zwischen 0,1 - 10 mm, am besten 1 - 5 mm, erfolgt.

4. Verfahren nach einem der vorhergehenden Ansprüche, wobei das Hydrokolloid, das mit Metallkationen geliert, Natriumalginat ist.

5. Verfahren nach einem der vorhergehenden Ansprüche, wobei die wässrige Lösung eines Metallkations mit einer Valenz von mindestens 2 lösliche Kalzium- oder Magnesiumsalze oder Mischungen davon enthält.

6. Verfahren nach einem der vorhergehenden Ansprüche, wobei die Konzentration des Metallkations in der wässrigen Lösung konstant gehalten wird.

7. Verfahren nach einem der vorhergehenden Ansprüche, wobei das Hydrokolloid, das mit Metallkationen geliert, in einer Menge von 0,5 - 20 Gew.-%, vorzugsweise von 2 - 10 Gew.-%, am besten von 4,5 - 5 Gew.-% basierend auf dem Gewicht der Larven vorhanden ist.

8. Verfahren nach einem der vorhergehenden Ansprüche, wobei der Protein-Hydrokolloidschlamm aus 10 - 75 Gew.-% Larven, bevorzugter aus 20 - 50 Gew.-%, noch bevorzugter aus 30 - 40 Gew.-%, basierend auf dem kombinierten Gewicht von Larven, Wasser und Hydrokolloid, besteht.

9. Verfahren nach einem der vorhergehenden Ansprüche, wobei die wässrige Lösung eines Metallkations mit einer Valenz von mindestens 2 als Bad vorhanden ist und die flüssige Oberfläche der wässrigen Lösung die Badoberfläche ist, oder wobei die wässrige Lösung eines Metallkations mit einer Valenz von mindestens 2 als fallender Film vorhanden ist und die flüssige Oberfläche der wässrigen Lösung die Oberfläche des fallenden Films ist.

10. Verfahren nach einem der vorhergehenden Ansprüche, wobei die Flocken des essbaren Proteinprodukts von der wässrigen Lösung getrennt werden.

11. Verfahren nach einem der vorhergehenden Ansprüche, wobei die Flocken des essbaren Proteinprodukts mit Wasser gespült werden, um überschüssige Metallkationen zu entfernen.

12. Verfahren nach einem der vorhergehenden Ansprüche, wobei die gespülten Flocken des essbaren Proteinprodukts, vorzugsweise mit einer Entwässerungspresse, vorzugsweise einer Entwässerungsschneckenpresse, und vorzugsweise auf einen Feuchtegehalt von 50 - 90 Gew.-%, vorzugsweise 60 - 90 Gew.-%, entwässert werden.

13. System (1) zur kontinuierlichen Herstellung eines strukturierten essbaren Proteinprodukts aus Insektenlarven oder -würmern, umfassend:
- ein Reaktionsgefäß (15) zum Halten einer wässrigen Lösung eines Metallkations mit einer Valenz von mindestens 2,
- ein Reservoir (8) zum Halten einer Protein-Hydrokolloidschlamms, wobei das Reservoir (8) einen Auslass (24) in Fluidkommunikation mit einer Pumpe (16) und Düse (18) aufweist, der so eingerichtet ist, dass er einen unter Druck stehenden Strom des Protein-Hydrokolloidschlamms von außerhalb der wässrigen Lösung eines Metallkations mit einer Valenz von mindestens 2 in die wässrige Lösung in einem Schrägwinkel in Bezug auf eine flüssige Oberfläche der wässrigen Lösung injiziert,
- ein kontrolliertes Zufuhrmittel (25) zur Zufuhr von Metallkation in das Reaktionsgefäß (15) zur Aufrechterhaltung einer konstanten Konzentration des Metallkation in der wässrigen Lösung,
- einen Separator (19) zum Trennen des essbaren Proteinprodukts von der wässrigen Lösung,
- eine Spüleinheit (22) zum Spülen des abgeschiedenen essbaren Proteinprodukts,
- eine Entwässerungspresse (23) zum Entwässern und Verdichten des abgespülten essbaren Proteinprodukts.

14. System nach Anspruch 13, wobei der Auslass (24) in Fluidkommunikation mit mehreren Kombinationen von Pumpen und Düsen steht, um mehrere unter Druck stehende Ströme des Protein-Hydrokolloidschlamms gleichzeitig zu injizieren.

## Revendications

1. Procédé de production d'un produit protéique comestible texturé dérivé de larves d'insectes ou de vers, le procédé comportant les étapes suivantes :
a) la réduction de la taille des larves d'insectes ou des vers pour obtenir une pâte,
b) le mélange de la pâte à un hydrocolloïde qui se gélifie avec des cations métalliques dans une solution aqueuse pour former une suspension protéique-hydrocolloïde,
c) l'injection de la suspension protéique-hydrocolloïde dans une solution aqueuse d'un cation métallique ayant une valence d'au moins 2 pour former le produit protéique comestible texturé,
dans lequel lors de l'injection à l'étape c), la suspension protéique-hydrocolloïde est projetée sous pression depuis l'extérieur de la solution aqueuse d'un cation métallique ayant une valence d'au moins 2 dans la solution aqueuse à un angle oblique par rapport à une surface liquide de la solution aqueuse, pour ainsi obtenir des flocons du produit protéique comestible texturé dans la solution aqueuse, flocons dans lesquels la quasi-totalité de l'hydrocolloïde a été gélifiée par les cations métalliques.

2. Procédé selon la revendication 1, dans lequel l'angle oblique a une valeur comprise entre 10 et 60°, de préférence entre 15 et 50°, de manière davantage préférée entre 20 et 40°.

3. Procédé selon la revendication 1 ou 2, dans lequel la projection sous pression est réalisée à travers une ouverture, de préférence une buse, de manière davantage préférée une buse d'un diamètre compris entre 0,1 et 10 mm, de manière préférée entre toutes entre 1 et 5 mm.

4. Procédé selon l'une quelconque des revendications précédentes, dans lequel l'hydrocolloïde qui se gélifie avec des cations métalliques est de l'alginate de sodium.

5. Procédé selon l'une quelconque des revendications précédentes, dans lequel la solution aqueuse d'un cation métallique ayant une valence d'au moins 2 contient des sels solubles de calcium ou de magnésium ou leurs mélanges.

6. Procédé selon l'une quelconque des revendications précédentes, dans lequel la concentration en cation métallique dans la solution aqueuse est maintenue constante.

7. Procédé selon l'une quelconque des revendications précédentes, dans lequel l'hydrocolloïde qui se gélifie avec des cations métalliques est présent en une quantité de 0,5 à 20 % en poids, de préférence de 2 à 10 % en poids, de manière préférée entre toutes de 4,5 à 5 % en poids sur la base du poids des larves.

8. Procédé selon l'une quelconque des revendications précédentes, dans lequel la suspension protéique-hydrocolloïde comporte de 10 à 75 % en poids de larves, de manière davantage préférée de 20 à 50 % en poids, de manière encore davantage préférée de 30 à 40 % en poids, sur la base du poids combiné de larves, d'eau et d'hydrocolloïde.

9. Procédé selon l'une quelconque des revendications précédentes, dans lequel la solution aqueuse d'un cation métallique ayant une valence d'au moins 2 est présente sous forme de bain et la surface liquide de la solution aqueuse est la surface du bain, ou dans lequel la solution aqueuse d'un cation métallique ayant une valence d'au moins 2 est présente sous forme de film tombant, et la surface liquide de la solution aqueuse est la surface du film tombant.

10. Procédé selon l'une quelconque des revendications précédentes, dans lequel les flocons du produit protéique comestible sont séparés de la solution aqueuse.

11. Procédé selon l'une quelconque des revendications précédentes, dans lequel les flocons du produit protéique comestible sont rincés à l'eau pour éliminer les cations métalliques en excès.

12. Procédé selon l'une quelconque des revendications précédentes, dans lequel les flocons rincés du produit protéique comestible sont déshydratés, de préférence avec une presse essoreuse, de manière davantage préférée une presse essoreuse à vis, et de préférence à une teneur en humidité de 50 à 90 % en poids, de manière davantage préférée de 60 à 90 % en poids.

13. Système (1) de production continue d'un produit protéique comestible texturé dérivé de larves d'insectes ou de vers, le système comportant :
- un récipient à réaction (15) pour contenir une solution aqueuse d'un cation métallique ayant une valence d'au moins 2,
- un réservoir (8) pour contenir une suspension protéique-hydrocolloïde, ledit réservoir (8) présentant une sortie (24) en communication fluidique avec une pompe (16) et une buse (18) configurée pour injecter un flux sous pression de la suspension protéique-hydrocolloïde depuis l'extérieur de la solution aqueuse d'un cation métallique ayant une valence d'au moins 2 dans la solution aqueuse à un angle oblique par rapport à une surface liquide de la solution aqueuse,
- un moyen d'alimentation commandé (25) pour alimenter le récipient à réaction (15) en cation métallique afin de maintenir une concentration constante en cation métallique dans la solution aqueuse,
- un séparateur (19) pour séparer le produit protéique comestible de la solution aqueuse,
- une unité de rinçage (22) pour rincer le produit protéique comestible séparé,
- une presse essoreuse (23) pour essorer et compacter le produit protéique comestible rincé.

14. Système selon la revendication 13, dans lequel la sortie (24) est en communication fluidique avec de multiples combinaisons de pompes et de buses, pour injecter simultanément de multiples flux sous pression de la suspension protéique-hydrocolloïde.
